# EUROPEAN PATENT APPLICATION

(11) **EP 3 162 276 A1**
(43) Date of publication of application: **03.05.2017**
(21) Application number: 15815298.3
(22) Date of filing: 20.03.2015
(51) Int. Cl.: A61B 1/12, G06Q 50/22

(54) **SYSTEM AND METHOD FOR MANAGING ENDOSCOPIC SCOPE**

(30) Priority: 30.06.2014 KR 20140080735
(71) Applicant: Choi, Dae Myung, Seongnam-si, Gyeonggi-do 461-809 (KR); Choi, Dong Yeol, Seongnam-si, Gyeonggi-do 461-809 (KR)
(72) Inventor: Choi, Dae Myung, Seongnam-si, Gyeonggi-do 461-809 (KR); Choi, Dong Yeol, Seongnam-si, Gyeonggi-do 461-809 (KR)
(74) Representative: Arth, Hans-Lothar
(86) International application number: PCT/KR2015/002759
(87) International publication number: WO 2016/003052

(57) **Abstract**

The present invention relates to a system and method for managing an endoscopic scope, the system comprising: multiple endoscope test devices (21) including an endoscopic scope (212) having an RFID chip (211) for storing endoscopic scope identification (ID) data embedded therein, an RFID reader (216) for reading the endoscopic scope ID data, and an endoscope control unit (213) for receiving the endoscopic scope ID data from the RFID reader (216), outputting the endoscopic scope ID data to an endoscope management server (10), and receiving record data of the endoscope test devices (21); multiple disinfection devices (32) comprising disinfection devices (32) including an RFID reader (322) for reading the endoscopic scope ID data from the RFID chip (211) of the endoscopic scope (212), the multiple disinfection devices (32) including RFID readers (322) for reading the endoscopic scope ID data from the RFID chip (211) mounted on the disinfected endoscopic scope (212) respectively and a disinfection irrigation control unit (321) for receiving, from the RFID readers (322), and transmitting, to an endoscope management server (10), the endoscopic scope ID data; and a leakage test device (40) configured to read the endoscopic scope ID data from the RFID chip (211) of the endoscopic scope (212) and output leakage test information to the endoscope management server (10), thereby efficiently managing sanitation with regard to disinfection and irrigation for each of endoscope test rooms, endoscope test devices, disinfection rooms, and leakage test devices, and achieving thorough disinfection and sanitary management of an endoscopic scope.

## Description

### Technical Field

The present invention relates to a system and method for managing an endoscopic scope, and more particularly, to a system and method for managing an endoscopic scope capable of stably performing cleaning and disinfection management after use of an endoscopic scope for a test by enabling an endoscope test device and an endoscope disinfection device to read identification information of an endoscopic scope from an RFID chip installed in the endoscopic scope to receive endoscope management information on the endoscopic scope test and endoscopic scope disinfection and update the same and to transmit the updated information to an endoscope management server and store the information in an endoscope management database.

### Background Art

An endoscope is inserted into a living body such as a human body, and is used in diagnosing or treating an organ, or collecting a specimen. The endoscope is repeatedly used for multiple patients. For this reason, it is necessary to thoroughly perform hygienic management of the endoscope after use, and it is necessary to thoroughly clean and disinfect the endoscope after each use, in order to completely prevent bacterial infection from occurring via the endoscope.

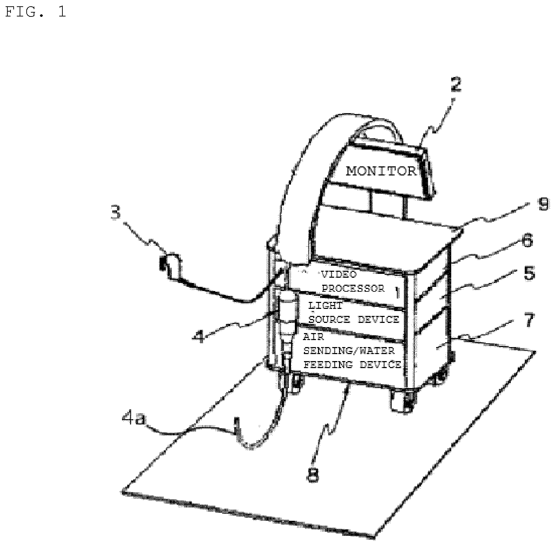

FIG. 1 shows the exterior of an endoscope system.

As shown in FIG. 1, the endoscope system includes a video processor 6, an air sending/water feeding device 7, and a monitor 2 as well as a light source device 5 for supplying light to an endoscope 4. The video processor 6 is configured to process an image capture signal captured by an image capture device (not shown) mounted on the endoscope 4 to generate an endoscopic image. In addition, the air sending/water feeding device 7 is configured to perform air blowing, water feeding, and suction for the endoscope 4.

The monitor 2 is a display means such as, for example, a liquid crystal monitor for displaying an endoscopic image generated by the video processor 6. Further, a remote controller (hereinafter, referred to as "remote control") 3 may be detachably connected to the video processor 6.

By manipulating the remote control 3 by the operator, control of the bending operation of the bending piece of the endoscope 4, which will be described later, and image control such as display of an image captured by the endoscope 4 may be easily performed during the body cavity test by the endoscope 4.

The endoscope 4 is used for test or treatment of the body cavity of a subject. The endoscope 4 generally has a tubular insertion portion 4a which is inserted into a body cavity and various endoscope conduits (channels) are formed in the insertion portion 4a in the axial direction of the insertion portion.

The endoscope conduits include, for example, a suction conduit. Thereby, once the insertion portion 4a of the endoscope is inserted into the body cavity, contaminants such as bodily fluids adhere not only to the outer surface of the insertion portion 4a but also to the inside of the insertion portion 4a, that is, the endoscope conduits (channels). Therefore, after use, the outer surface of the insertion portion 4a and the endoscope channels of the endoscope 4 need to be sufficiently cleaned and disinfected.

When the frequency of use of the endoscope is high, a plurality of endoscopes is used, and cleaning and disinfection thereof are sequentially performed. However, cleaning and disinfection of the endoscopes requires considerable time. In particular, regarding disinfection, the type of disinfectant and the immersion time for the disinfectant are determined depending on a testee's medical history.

A management system in which information on the end time of the endoscopic test is updated every time the endoscopic test is completed has been proposed and includes an acquisition unit for acquiring information on the end time of the latest endoscopic test performed in the endoscopic system, and a management unit for managing the state of cleaning and disinfection performed by an endoscope cleaning/disinfecting apparatus in accordance with the information (See Patent Document 1).

There is provided an apparatus for cleaning and disinfection of endoscopes which includes an individual cleaning and disinfecting chamber independently provided in the body of the cleaning and disinfecting apparatus and which is capable of performing cleaning or disinfection by operating each group independently or by operating a plurality of groups at the same time (See Patent Document 2).

### Disclosure

### Technical Problem

Therefore, the present invention has been made in view of the above problems, and it is one object of the present invention to provide a system for managing an endoscopic scope capable of preventing and managing damage of an endoscope caused by prevention of infection by repeated use of the endoscope and repeated sterilization of the endoscope, and a method for managing the same.

It is another object of the present invention to provide a system for managing an endoscopic scope that acquires the end time of the latest endoscopic test executed in the endoscope system, sets the time of cleaning and disinfection performed in the endoscope cleaning/disinfecting apparatus in proportion to the acquired end time, and updates the end time of an endoscopic test every time the endoscopic test ends, and a method for managing the same.

It is still another object of the present invention to provide a system for managing an endoscopic scope which is capable of obtaining a good and stable cleaning result regardless of the amount of time between completion of a test and cleaning by allowing the time for disinfection of the endoscope to be prolonged according to the amount of time between completion of the test and cleaning, and a method for managing the same.

### Technical Solution

In accordance with one aspect of the present invention, provided is a management system for an endoscopic scope including: a plurality of endoscope test devices installed in a plurality of endoscopic test rooms and each including an RFID reader for reading endoscopic scope identification (ID) data in the endoscopic scope provided with an RFID chip having the endoscopic scope ID data stored therein, the endoscope test devices being configured to transmit and receive endoscope management information to and from an endoscope management server and examine an internal organ or body cavity; a plurality of disinfection devices installed in a plurality of disinfection rooms and including an RFID reader for reading the endoscopic scope ID data in the RFID chip of the endoscopic scope, the disinfection devices being configured to transmit and receive endoscope management information to and from the endoscope management server and disinfect the endoscopic scope; a leakage test device including an RFID reader for reading the endoscopic scope ID data in the RFID chip of the endoscopic scope, the leakage test device being configured to transmit endoscope management information to the endoscope management server and check whether the endoscopic scope leaks to output a leakage test result to an endoscope management server; and the endoscope management server for receiving the endoscope management information including disinfection and leakage information on the endoscopic scope from the plurality of endoscope test devices, the plurality of disinfection devices and the leakage test device and storing the same in an endoscope management database.

According to an embodiment of the present invention, the RFID chip of the endoscopic scope stores the endoscopic scope ID data for identifying the endoscopic scope, wherein the endoscopic scope ID data includes a model name, an endoscope manufacturing date, and an endoscope serial number.

According to an embodiment of the present invention, the endoscope control units of the endoscope test devices transmit the endoscopic scope ID data to the endoscope management server, receive and update endoscopic test history data of the endoscope test devices corresponding to the endoscopic scope ID data from the endoscope management server, and transmit the updated endoscopic test history data to the endoscope management server to store the transmitted endoscopic test history data in the endoscope management database.

According to an embodiment of the present invention, each of the endoscope test devices includes: an RFID chip storing the endoscopic scope ID data and embedded in the endoscopic scope; the endoscopic scope including the RFID chip; a communication unit for communicating with the endoscope management server; and an endoscope control unit configured to control each part of the endoscope test device, transmit and receive the endoscopic scope ID data, endoscope specific data and test data to and from a test room control unit via the communication unit, receive an image of an internal organ or body cavity through the endoscopic scope, process the received image, read the endoscopic scope ID data from the RFID reader and transmit the endoscopic scope ID data to the test room control unit through the communication unit; and an endoscope memory unit for storing the unique data of the endoscope test device and the test data.

According to an embodiment of the present invention, the unique data of the endoscope test device includes the number of times of use, a repair history, a failure history, a receipt time, and an expected disposal time, and the test data includes a test date, a test start time, a test end time, data of instruments used, and a test conductor.

According to an embodiment of the present invention, after performing a leakage test, the leakage test device reads the endoscopic scope ID data of the endoscopic scope, transmits the endoscopic scope ID data and a leakage test result together to the endoscope management server to store the endoscopic scope ID data and the leakage test result in an endoscope test device database and a leakage test database in the endoscope management database.

According to an embodiment of the present invention, the disinfection/cleaning control unit determines a cleaning time and a disinfection time of the endoscopic scope based on the test data of the endoscopic scope, testee data, and cleaning/disinfection history data received from the endoscope management server to control the disinfection device to perform cleaning and disinfection.

According to an embodiment of the present invention, the cleaning time and the disinfection time of the endoscopic scope are determined according to a most recent test time in the test data of the endoscopic scope and a tested disease name in the testee data.

According to an embodiment of the present invention, the endoscope management database includes an endoscope test device database for storing endoscope specific data containing the number of times of use, repair history information and CCD characteristic data and test data containing a date of a test, a test start time, a test end time, data of an instrument used and a test conductor; a disinfection device database for storing cleaning/disinfection history data containing a date of cleaning and disinfection, a cleaning/disinfection end time, the number of times of the cleaning and disinfection, information on a cleaning/disinfection device used, and a conductor performing the cleaning and disinfection; leakage test data for storing a result of checking whether the endoscopic scope leaks; a testee database containing a sex, name, and age of a testee, a tested portion, a tested disease name, and a test time; and an endoscopic scope database including endoscope specific data containing the number of times of use of the endoscopic scope and repair history information and test data containing a date of the test, a test start time, a test end time, data of an instrument used, and a test conductor.

In accordance with another aspect of the present invention, provided is a method for managing an endoscopic scope including: an endoscope control unit of an endoscope test device reading endoscopic scope identification (ID) data in an RFID chip of an endoscopic scope, transmitting the endoscopic scope ID data to an endoscope management server, and receiving history data of the endoscope test device and the endoscopic scope from the endoscope management server; the endoscope control unit of the endoscope test device updating endoscopic test data and unique data, storing the updated endoscopic test data and unique data in an endoscope memory unit, and storing the updated endoscopic test data in an endoscope test device database by transmitting the updated endoscopic test data to the endoscope management server; a leakage test control unit of a leakage test device performing a leakage test on the endoscopic scope, reading the endoscope ID data and sending the same to the endoscope management server along with a result of the leakage test, and storing the result and the endoscope ID data in a leakage test database and an endoscopic scope database; when the endoscopic scope used for a endoscopic test is cleaned and disinfected in a disinfection room, a disinfection/cleaning control unit of a disinfection device reading the endoscopic scope ID data and transmitting the endoscopic scope ID data to the endoscope management server, and the endoscope management server transmitting test data of the endoscopic scope, testee data, and cleaning/disinfection history data to the disinfection/cleaning control unit; the disinfection/cleaning control unit determining a cleaning time and a disinfection time of the endoscopic scope based on the test data of the endoscopic scope, the testee data, and the cleaning/disinfection history data; and when the cleaning and disinfection of the endoscopic scope is completed, the disinfection/cleaning control unit updating the cleaning/disinfection history data, transmitting the cleaning/disinfection history data along with the endoscope ID data to the endoscope management server, and storing the data in an endoscope management database.

### Advantageous Effects

As apparent from the foregoing description, according to the present invention, it is possible to manage endoscope management data per endoscopic test room, endoscope test device, disinfection room, and leakage inspection device, thereby efficiently managing hygiene management of cleaning and disinfection of the endoscope test devices. In addition, it is possible to implement thorough disinfection and sanitization by determining the cleaning time and disinfection time of an endoscopic scope according to the most recent test time and the tested disease name in the testee data.

### Description of Drawings

FIG. 1 is a perspective view showing the exterior of an endoscope system according to the prior art.
FIG. 2 is a block diagram illustrating the configuration of a management system for an endoscopic scope according to the present invention.
FIG. 3 is a block diagram illustrating the configuration of an endoscope test device installed in an endoscopic test room according to an embodiment of the present invention.
FIG. 4 is a block diagram illustrating the configuration of an embodiment of a leakage test device according to the present invention.
FIG. 5 is a block diagram illustrating the configuration of an embodiment of a disinfection device installed in a disinfection room according to the present invention;
FIG. 6 is a block diagram illustrating the configuration of an embodiment of an endoscope management database according to the present invention;
FIG. 7 is a flowchart illustrating a method for managing an endoscopic scope according to the present invention.

### Best mode

Hereinafter, preferred embodiments of the present invention will be described in detail with reference to the accompanying drawings.

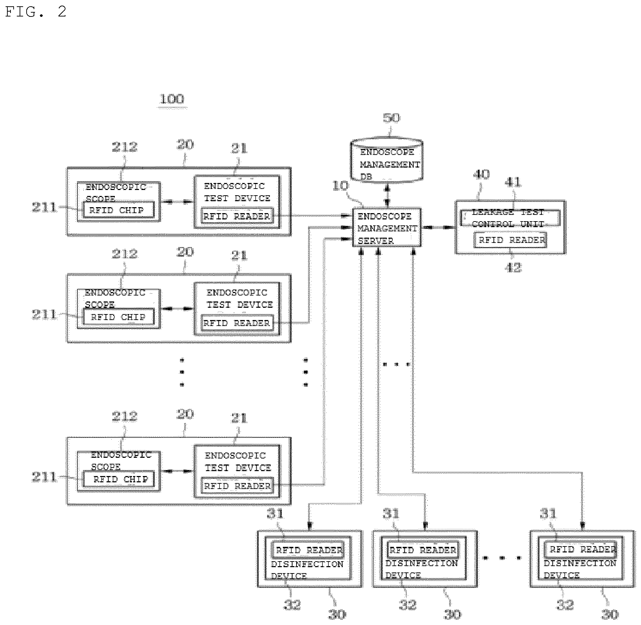

FIG. 2 is a block diagram illustrating the configuration of a management system for an endoscopic scope according to the present invention.

A management system 100 for an endoscopic scope according to the present invention includes: a plurality of endoscope test devices 21 installed in a plurality of endoscopic test rooms 20 and including an RFID reader 216 for reading endoscopic scope identification (ID) data in an endoscopic scope 212 provided with an RFID chip 211 having the endoscopic scope ID data stored therein, the endoscope test devices 21 being configured to transmit and receive endoscope management information along with the endoscopic scope ID data to and from an endoscope management server 10 and to examine an internal organ or body cavity; a plurality of disinfection devices 32 installed in a plurality of disinfection rooms 30 and including an RFID reader 322 for reading the endoscopic scope ID data in the RFID chip 211 of the endoscopic scope 212, the disinfection devices 32 being configured to transmit and receive endoscope management information along with the endoscopic scope ID data to and from the endoscope management server 10 and disinfect the endoscopic scope 212; a leakage test device 40 including an RFID reader 42 for reading the endoscopic scope ID data in the RFID chip 211 of the endoscopic scope 212, the leakage test device 40 being configured to transmit endoscope management information along with the endoscopic scope ID data to the endoscope management server 10 and check whether the endoscopic scope 212 leaks to transmit a leakage test result to the endoscope management server 10; and the endoscope management server 10 for receiving the endoscope management information including disinfection and leakage information on each of the endoscopic scopes 212 from the plurality of endoscope test devices 21, the plurality of disinfection devices 32 and the leakage test device 40 and storing the same in an endoscope management database 50.

The RFID chip 211 of the endoscopic scope 212 stores the endoscopic scope ID data for identifying the endoscopic scope 212. The endoscopic scope ID data is read by the RFID reader 216 of the endoscope test devices 21, input to an endoscope control unit 213 of the endoscope test devices 21, and transmitted along with the endoscope management information to the endoscope management server 10 via the communication unit 214.

Herein, the endoscopic scope 212 takes the form of a tube inserted into the human body, and the endoscopic scope ID data includes a model name, an endoscope date of manufacture, and an endoscope serial number.

The endoscope test device 21 receives the endoscopic scope ID data from the RFID reader 216 and transmits the endoscope management information along with the endoscopic scope ID data to the endoscope management server 10, and the endoscope management server 10 updates the endoscopic test history data corresponding to the endoscopic scope 212 in the endoscopic scope database 56 with the endoscopic scope ID data.

The endoscopic test history data may include a date of a test, a start time of the test, an end time of the test, data of the instrument used, and the test conductor.

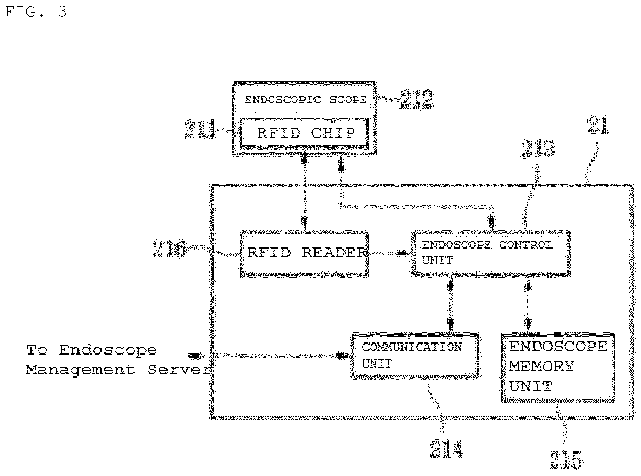

FIG. 3 is a block diagram illustrating the configuration of an endoscope test device installed in an endoscopic test room according to an embodiment of the present invention.

The endoscope test device 21 according to the present invention includes an RFID chip 211 storing endoscopic scope ID data and embedded in the endoscopic scope 212; the endoscopic scope 212 including the RFID chip 211 and inserted into the human body; a communication unit 214 for communicating with the endoscope management server 10; an RFID reader 216 for reading the endoscopic scope ID data in the RFID chip 211 embedded in the endoscopic scope 212; an endoscope control unit 213 configured to control each part of the endoscope test device 21, transmit and receive the endoscope management information to and from the endoscope management server 10 via the communication unit 214, receive an image of an interior of an internal organ or body cavity through the endoscopic scope 212, process the received image, read the endoscopic scope ID data from the RFID reader 216 and transmit the ID data to the endoscope management server through the communication unit 214; and an endoscope memory unit 215 for storing unique data (use frequency, repair history, failure history, a receipt time, expected disposal time, etc.) of the endoscope test device 21 and the test data (test date, test start time, test end time, data of instruments used, test conductor, etc.).

The endoscopic scope 212 inserted into the internal organs or body cavity is removed from the endoscope test device 21 so as to be cleaned and disinfected, and is thus indicated as a separate block. The endoscope test device 21 further includes a light source device for providing a light source to the endoscopic scope 212, an air sending/water feeding device for supplying air and water to the endoscopic scope 212, and a monitor for displaying an image captured by the endoscopic scope 212, but description of parts aside from the endoscope management information is omitted for the sake of simplicity of explanation.

The endoscope control unit 213 receives the unique data (repair history, failure history, receipt time, expected disposal time, etc.) and the test data (the test execution date, the test start time, the test end time, data of an instrument used, the test conductor, etc.) of the endoscope test device 21 and the endoscopic scope 212 through the communication unit 214 and transmits the updated history data of the endoscope test device 21 and the endoscopic scope 212 to the endoscope management server 10.

The endoscope management server 10 receives the updated history data of the endoscope test device 21 and the endoscopic scope 212 and updates the endoscope management database 50 by storing the received data in the endoscope test device database 51 and the endoscopic scope database 56 of the endoscope management database 50.

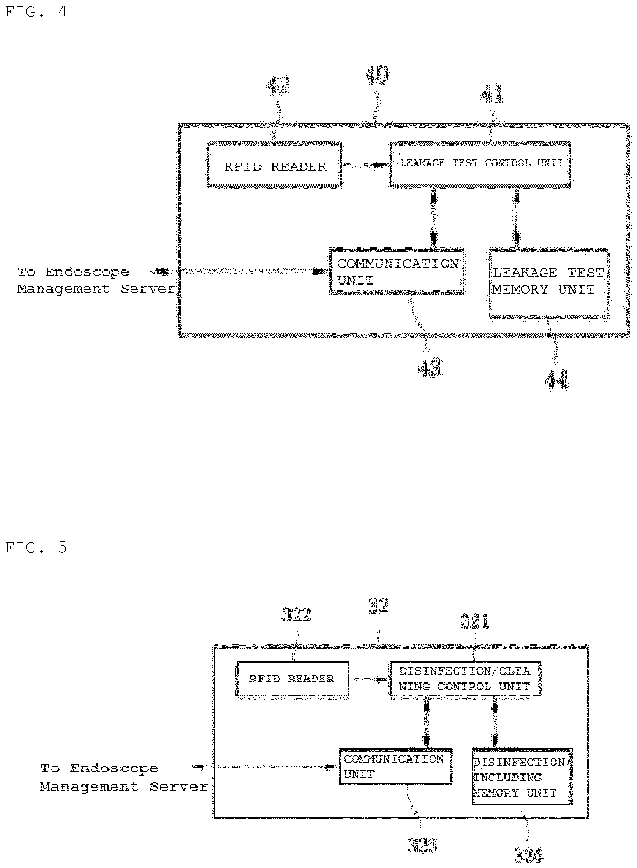

FIG. 4 is a block diagram illustrating the configuration of an embodiment of a leakage test device according to the present invention.

The leakage test apparatus 40 according to the present invention includes: a communication unit 43 for transmitting and receiving leakage test data (test execution date, test start time, test end time, data of instruments used, test conductor, etc.) of the endoscope management server 10 and the endoscopic scope 212; an RFID reader 42 for reading the endoscopic scope ID data in the RFID chip 211 embedded in the endoscopic scope 212; a leakage test control unit 41 for controlling each part of the leakage test device 40, reading the endoscopic scope ID data from the RFID reader 216 via the communication unit 43 and transmitting the endoscopic scope ID data along with along with leakage test data of the endoscopic scope 212 to the endoscope management server 10; and a leakage test memory unit 44 for storing the leakage test data (test execution date, test start time, test end time, data of an instrument used, test conductor, etc.) of the leakage test device 40.

Before the endoscope test device 21 is used for the procedure, the leakage test device 40 performs a leakage test. After the leakage test is performed, the leakage test control unit 41 reads the endoscopic scope ID data stored in the RFID chip 211 of the endoscopic scope 212 and transmits the data along with the leakage test result to the endoscope management server 10, such that the endoscopic scope ID data and the leakage test result are stored together in the endoscope test device database 51 and the leakage test database 54 of the endoscope management database 50.

The endoscope test device 21 having passed the leakage test is used for the endoscopic test. When the test is completed, the endoscopic scope 212 is separated from the endoscope test device 21 used for the test and cleaned and disinfected in the disinfection room 30.

FIG. 5 is a block diagram illustrating the configuration of an embodiment of a disinfection device installed in a disinfection room according to the present invention.

The endoscopic scope ID data is wirelessly read by the RFID reader 322 while the endoscopic scope 212 is brought close to the RFID reader 322 of the disinfection device 32 at the time of cleaning and disinfection in the disinfection room 30. The RFID reader 322 reads the disinfector ID data (e.g., name, affiliation, etc.) attached to a disinfector in the disinfection room 30 and inputs the data to a disinfection/cleaning control unit 321.

The disinfection/cleaning control unit 321 sends the endoscopic scope ID data and the disinfector ID data to the endoscope management server 10. The endoscope management server 10 stores the endoscopic scope ID data and the disinfector ID data in a disinfection device database 53, retrieves the test data (test execution date, test start time, test end time, data of an instrument used, test conductor, etc.) of the endoscopic scope 212, testee data (sex, name, age, tested portion, tested disease name, test time, etc.) corresponding to the endoscopic scope ID data from the endoscopic scope database 56 and the testee database 55, and retrieves cleaning/disinfection history data (the date of cleaning and disinfection, the end time of cleaning and disinfection, the number of times of cleaning/disinfection, information on the cleaning/disinfection device used, the cleaning disinfectant, the conductor of cleaning/disinfection, etc.) corresponding to the disinfection device 32 from the disinfection device database 53 of the endoscope management database 50.

The endoscope management server 10 transmits the retrieved test data of the endoscopic scope 212 and the retrieved data to the disinfection/cleaning control unit 321 along with the cleaning/disinfection history data and the endoscopic scope ID data.

The disinfection/cleaning control unit 321 determines the cleaning time and disinfection time of the endoscopic scope 212 based on the test data of the corresponding endoscopic scope 212, the testee data and the cleaning/disinfection history data to control the disinfection device 30 to perform cleaning and disinfecting.

At this time, the cleaning time and disinfection time of the endoscopic scope 212 may be determined according to the most recent test time in the test data of the endoscopic scope 212 and a tested disease name in the testee data.

When cleaning and disinfection of the endoscopic scope 212 are completed, the cleaning/disinfection history data of the endoscopic scope 212 is updated and transmitted to the endoscope management server 10 along with the corresponding endoscopic scope ID data. The endoscope management server 10 updates and stores the updated cleaning/disinfection history data in the disinfection device database 53 and the endoscopic scope database 56 of the endoscope management database 50 along with the endoscopic scope identification (ID) data.

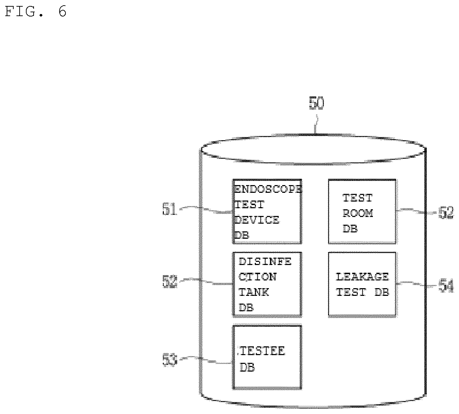

FIG. 6 is a block diagram illustrating the configuration of an embodiment of an endoscope management database according to the present invention.

The endoscope management database 50 according to the present invention includes: an endoscope test device database 51 for storing endoscope specific data containing the number of times of use, repair history information and CCD characteristic data and test data containing a date of a test, a test start time, a test end time, data of an instrument used and a test conductor; a disinfection device database 53 for storing cleaning/disinfection history data containing a date of cleaning and disinfection, a cleaning/disinfection end time, the number of times of cleaning and disinfection, information on a cleaning/disinfection device used, and a conductor performing cleaning and disinfection; leakage test data 54 for storing a result of checking whether the endoscopic scope 212 leaks; a testee database 55 containing a sex, name and age of a testee, a tested portion, a tested disease name, and a test time; and an endoscopic scope database 56 including endoscope specific data containing the number of times of use of the endoscopic scope 212 and repair history information and test data containing a test date, a test start time, a test end time, data of an instrument used, and a test conductor.

The data stored in the endoscope management database 50 according to the present invention is stored along with the endoscopic scope ID data. Accordingly, the endoscope specific data, the test data, the cleaning/disinfection history data, and the testee data may be retrieved using the endoscopic scope ID data.

Therefore, the endoscope management data can be managed by the endoscope test device, disinfection device, and leakage test device, and accordingly hygienic management of cleaning and disinfection of endoscope test devices may be efficiently performed.

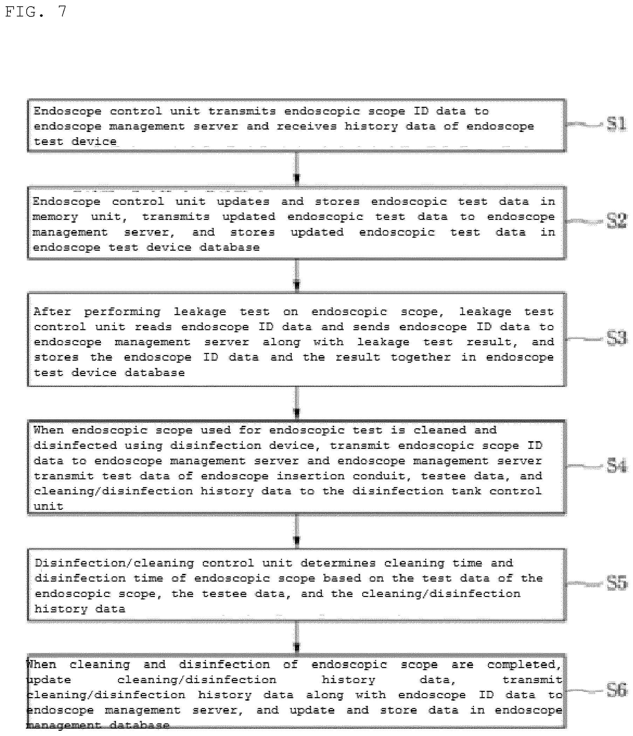

FIG. 7 is a flowchart illustrating a method for managing an endoscopic scope according to the present invention.

In step S1, the endoscopic control unit 213 of the endoscope test device 21 reads the endoscopic scope ID data contained in the RFID chip 211 of the endoscopic scope 212 and transmits the same to the endoscope management server 10, and receives the history data of the endoscope test device 21 and the endoscopic scope 212 from the endoscope management server 10.

In step S2, the endoscope control unit 213 of the endoscope test device 21 updates endoscopic test data (test date, test start time, test end time, data of instruments used, test conductor, etc.) and unique data (use frequency, repair history, failure history, a receipt time, expected disposal time, etc.) and stores the updated data in the endoscope memory unit 215, and transmits the updated endoscopic test data to the endoscope management server 10 to store the updated endoscopic test data in the endoscope test device database 51.

In step S3, after the leakage test control unit 41 of the leakage test device 40 performs a leakage test on the endoscopic scope 212, the leakage test control unit 41 reads the endoscopic scope ID data of the endoscopic scope 212, transmits the same to the endoscope management server 10 along with the leakage test result, and stores the leakage test result and the endoscopic scope ID data in the leakage test database 54 and the endoscopic scope database 56.

In step S4, in cleaning and disinfecting the endoscopic scope 212 used for the endoscopic test in the disinfection room 30, the disinfection/cleaning control unit 321 of the disinfection device 30 reads the endoscopic scope ID data contained in the RFID chip 211 of the endoscopic scope 212 and transmits the same to the endoscope management server 10, and the endoscope management server 10 transmits the test data of the endoscopic scope 212, the testee data and the cleaning/disinfection history data to the disinfecting room 30.

In step S5, the disinfection/cleaning control unit 321 of the disinfection room 30 determines the cleaning time and disinfection time of the endoscopic scope 212 based on the test data of the endoscopic scope 212, the testee data, and the cleaning/disinfection history data.

In step S6, when cleaning and disinfection of the endoscopic scope 212 are completed, the cleaning disinfection history data is updated and transmitted to the endoscope management server 10 along with the endoscopic scope ID data to store the data in the disinfection device database 53 and endoscopic scope database 56 of the endoscope management database 50.

It will be understood by those skilled in the art that various changes can be made therein without departing from the spirit and scope of the present invention as defined by the appended claims and their equivalents. Of course, the appended claims are intended to encompass the technical concepts forming such changes.

## Claims

1. A management system for an endoscopic scope comprising:
a plurality of endoscope test devices installed in a plurality of endoscopic test rooms and each comprising an RFID reader for reading endoscopic scope identification (ID) data in the endoscopic scope provided with an RFID chip having the endoscopic scope ID data stored therein, the endoscope test devices being configured to transmit and receive endoscope management information to and from an endoscope management server and examine an internal organ or body cavity;
a plurality of disinfection devices installed in a plurality of disinfection rooms and comprising an RFID reader for reading the endoscopic scope ID data in the RFID chip of the endoscopic scope, the disinfection devices being configured to transmit and receive endoscope management information to and from the endoscope management server and disinfect the endoscopic scope;
a leakage test device comprising an RFID reader for reading the endoscopic scope ID data in the RFID chip of the endoscopic scope, the leakage test device being configured to transmit endoscope management information to the endoscope management server and check whether the endoscopic scope leaks to output a leakage test result to an endoscope management server; and
the endoscope management server for receiving the endoscope management information including disinfection and leakage information on the endoscopic scope from the plurality of endoscope test devices, the plurality of disinfection devices and the leakage test device and storing the same in an endoscope management database.

2. The management system according to claim 1, wherein the RFID chip of the endoscopic scope stores the endoscopic scope ID data for identifying the endoscopic scope, wherein the endoscopic scope ID data comprises a model name, an endoscope manufacturing date, and an endoscope serial number.

3. The management system according to claim 1 or 2, wherein the endoscope control units of the endoscope test devices transmit the endoscopic scope ID data to the endoscope management server, receive and update endoscopic test history data of the endoscope test devices corresponding to the endoscopic scope ID data from the endoscope management server, and transmit the updated endoscopic test history data to the endoscope management server to store the transmitted endoscopic test history data in the endoscope management database.

4. The management system according to claim 1 or 2, wherein each of the endoscope test devices comprises: an RFID chip storing the endoscopic scope ID data and embedded in the endoscopic scope; the endoscopic scope including the RFID chip; a communication unit for communicating with the endoscope management server; and an endoscope control unit configured to control each part of the endoscope test device, transmit and receive the endoscopic scope ID data, endoscope specific data and test data to and from a test room control unit via the communication unit, receive an image of an internal organ or body cavity through the endoscopic scope, process the received image, read the endoscopic scope ID data from the RFID reader and transmit the endoscopic scope ID data to the test room control unit through the communication unit; and an endoscope memory unit for storing the unique data of the endoscope test device and the test data.

5. The management system according to claim 4, wherein the unique data of the endoscope test device comprises the number of times of use, a repair history, a failure history, a receipt time, and an expected disposal time, and the test data comprises a test date, a test start time, a test end time, data of instruments used, and a test conductor.

6. The management system according to claim 1 or 2, wherein, after performing a leakage test, the leakage test device reads the endoscopic scope ID data of the endoscopic scope, transmits the endoscopic scope ID data and a leakage test result together to the endoscope management server to store the endoscopic scope ID data and the leakage test result in an endoscope test device database and a leakage test database in the endoscope management database.

7. The management system according to claim 1 or 2, wherein the disinfection/cleaning control unit determines a cleaning time and a disinfection time of the endoscopic scope based on the test data of the endoscopic scope, testee data, and cleaning/disinfection history data received from the endoscope management server to control the disinfection device to perform cleaning and disinfection.

8. The system of claim 7, wherein the cleaning time and the disinfection time of the endoscopic scope are determinable according to a most recent test time in the test data of the endoscopic scope and a tested disease name in the testee data.

9. The endoscope management system according to claim 1, wherein the endoscope management database comprises an endoscope test device database for storing endoscope specific data containing the number of times of use, repair history information and CCD characteristic data and test data containing a date of a test, a test start time, a test end time, data of an instrument used and a test conductor; a disinfection device database for storing cleaning/disinfection history data containing a date of cleaning and disinfection, a cleaning/disinfection end time, the number of times of the cleaning and disinfection, information on a cleaning/disinfection device used, and a conductor performing the cleaning and disinfection; leakage test data for storing a result of checking whether the endoscopic scope leaks; a testee database containing a sex, name, and age of a testee, a tested portion, a tested disease name, and a test time; and an endoscopic scope database comprising endoscope specific data containing the number of times of use of the endoscopic scope and repair history information and test data containing a date of the test, a test start time, a test end time, data of an instrument used, and a test conductor.

10. A method for managing an endoscopic scope comprising:
an endoscope control unit of an endoscope test device reading endoscopic scope identification (ID) data in an RFID chip of an endoscopic scope, transmitting the endoscopic scope ID data to an endoscope management server, and receiving history data of the endoscope test device and the endoscopic scope from the endoscope management server;
the endoscope control unit of the endoscope test device updating endoscopic test data and unique data, storing the updated endoscopic test data and unique data in an endoscope memory unit, and storing the updated endoscopic test data in an endoscope test device database by transmitting the updated endoscopic test data to the endoscope management server;
a leakage test control unit of a leakage test device performing a leakage test on the endoscopic scope, reading the endoscope ID data and sending the same to the endoscope management server along with a result of the leakage test, and storing the result and the endoscope ID data in a leakage test database and an endoscopic scope database;
when the endoscopic scope used for a endoscopic test is cleaned and disinfected in a disinfection room, a disinfection/cleaning control unit of a disinfection device reading the endoscopic scope ID data and transmitting the endoscopic scope ID data to the endoscope management server, and the endoscope management server transmitting test data of the endoscopic scope, testee data, and cleaning/disinfection history data to the disinfection/cleaning control unit;
the disinfection/cleaning control unit determining a cleaning time and a disinfection time of the endoscopic scope based on the test data of the endoscopic scope, the testee data, and the cleaning/disinfection history data; and
when the cleaning and disinfection of the endoscopic scope is completed, the disinfection/cleaning control unit updating the cleaning/disinfection history data, transmitting the cleaning/disinfection history data along with the endoscope ID data to the endoscope management server, and storing the data in an endoscope management database.
